# EUROPEAN PATENT APPLICATION

(11) **EP 4 579 573 A1**
(43) Date of publication of application: **02.07.2025**
(21) Application number: 23856891.9
(22) Date of filing: 12.04.2023
(51) Int. Cl.: G06Q 50/10, G06Q 40/04

(54) **SYSTEM FOR CALCULATING CARBON DIOXIDE CONSUMPTION, METHOD OF CALCULATING CARBON DIOXIDE CONSUMPTION, AND SYSTEM FOR ASSISTING WITH CARBON DIOXIDE EMISSION TRADING**

(30) Priority: 26.08.2022 JP 2022134942
(71) Applicant: Niterra Co., Ltd., Nagoya-shi, Aichi 461-0005 (JP)
(72) Inventor: KAWASE Hiroki, Nagoya-shi, Aichi 461-0005 (JP); YANAGAWA Takayuki, Nagoya-shi, Aichi 461-0005 (JP); YASUDA Yutaka, Nagoya-shi, Aichi 461-0005 (JP)
(74) Representative: Grünecker Patent- und Rechtsanwälte PartG mbB
(86) International application number: PCT/JP2023/014869
(87) International publication number: WO 2024/042771

(57) **Abstract**

A system for calculating carbon dioxide consumption (70) is provided with: a first detection unit (71); a second detection unit (72); and a data processing device (75) (calculation unit). The first detection unit (71) is disposed on a first supply path (91) between: a supply unit (90) that is a supply source of carbon dioxide; and a carbon dioxide utilization device (80) for using the carbon dioxide supplied from the supply unit (90) as a starting material to produce a carbon dioxide-derived product. The first detection unit (71) detects a first parameter pertaining to the carbon dioxide supplied via the first supply path (91). The second detection unit (72) detects a second parameter pertaining to the carbon dioxide contained in the product generated at the carbon dioxide utilization device (80). On the basis of the first parameter and the second parameter, the data processing device (75) (calculation unit) calculates the amount of carbon dioxide consumed at the carbon dioxide utilization device (80).

## Description

### TECHNICAL FIELD

The present disclosure relates to a system for calculating the consumption amount of carbon dioxide (hereinafter referred to as a "carbon dioxide consumption amount calculation system"), a method for calculating the consumption amount of carbon dioxide (hereinafter referred to as a "carbon dioxide consumption amount calculation method"), and a system for supporting carbon dioxide emission trading (hereinafter referred to as a "carbon dioxide emission trading support system").

### BACKGROUND ART

In the method for trading carbon dioxide emission rights disclosed in Patent Literature 1, an emission right transferee calculates a carbon dioxide emission right to be generated by using an energy consuming apparatus and returns to a user of the energy consuming apparatus a compensation corresponding to the calculated carbon dioxide emission right. This trading method includes an apparatus ID recording step of recording in a management database, disposed on the emission right transferee, apparatus IDs respectively assigned to energy consuming apparatuses supplied from apparatus suppliers, and an emission right calculation step of calculating a carbon dioxide reduction amount from the energy consumption amount of the energy consuming apparatus of each apparatus ID and calculating, from the reduction amount, a carbon dioxide emission right which the user will obtain. In the trading method disclosed in Patent Literature 1, a CO₂ emission credit secured in advance by the emission right transferee is predictively calculated on the basis of the emission amount of CO₂ that can be reduced when an electric vehicle to be sold travels over an ordinary lifetime travel distance.

### CITATION LIST

### PATENT LITERATURE

Patent Literature 1: JP2011-134179A

### SUMMARY OF INVENTION

### TECHNICAL PROBLEM

The technique disclosed in Patent Literature 1 calculates the emission amount of CO₂ that can be reduced as a result of traveling of an electric vehicle, but does not relate to the concept of calculating the consumption amount in a "carbon dioxide utilization device which produces a carbon dioxide-originating product."

One object of the present invention is to provide a technique for more accurately calculating the amount of carbon dioxide consumed in a carbon dioxide utilization device which produces a carbon dioxide-originating product using carbon dioxide as a raw material.

### SOLUTION TO PROBLEM

A carbon dioxide consumption amount calculation system according to one mode of the present invention comprises:
a first detection section disposed in a raw material supply passage between a supply section serving as a carbon dioxide supply source and a carbon dioxide utilization device which produces a carbon dioxide-originating product by using, as a raw material, carbon dioxide supplied from the supply section, the first detection section detecting a first parameter related to carbon dioxide supplied via the raw material supply passage;
a second detection section which detects a second parameter related to carbon dioxide contained in the product produced by the carbon dioxide utilization device; and
a calculation section which calculates the consumption amount of carbon dioxide consumed in the carbon dioxide utilization device on the basis of the first parameter and the second parameter.

A carbon dioxide consumption amount calculation method according to another mode of the present invention comprises:
calculating the consumption amount of carbon dioxide consumed in a carbon dioxide utilization device on the basis of a first parameter and a second parameter related to carbon dioxide,
the first parameter being related to carbon dioxide supplied via a raw material supply passage between a supply section serving as a carbon dioxide supply source and the carbon dioxide utilization device which produces a carbon dioxide-originating product by using, as a raw material, carbon dioxide supplied from the supply section, and
the second parameter being related to carbon dioxide contained in the product produced by the carbon dioxide utilization device.

A carbon dioxide emission trading support system according to still another mode of the present invention comprises:
a management system having a token issuance section for issuing a token; and
a manager wallet associated with the management system,

wherein, in the case where a user account and a user wallet are associated with each other, and a consumption signal for notifying an amount of consumption of carbon dioxide is sent from a user terminal associated with the user account to the management system, the token issuance section issues the token on the basis of the consumption signal, and the management system instructs storage of the issued token in the user wallet, and
wherein the consumption signal is generated and sent on the basis of the consumption amount calculated by the above-described carbon dioxide consumption amount calculation method.

### ADVANTAGEOUS EFFECT OF INVENTION

The present invention makes it possible to more accurately calculate the amount of carbon dioxide consumed in a carbon dioxide utilization device which produces a carbon dioxide-originating product using carbon dioxide as a raw material.

### BRIEF DESCRIPTION OF DRAWINGS

[FIG. 1] FIG. 1 is an explanatory diagram used for describing a carbon dioxide utilization system to which a carbon dioxide consumption amount calculation system according to a first embodiment is applied, etc.
[FIG. 2] FIG. 2 is a schematic sectional view, showing, as an example, a reaction device which constitutes a portion of the carbon dioxide utilization system shown in FIG. 1.
[FIG. 3] FIG. 3 is an explanatory diagram schematically showing a trading system and a carbon dioxide emission trading support system to which the carbon dioxide consumption amount calculation system according to the first embodiment is applied.
[FIG. 4] FIG. 4 is an explanatory diagram used for describing the trading system of FIG. 3 from a point of view different from FIG. 3.
[FIG. 5] FIG. 5(A) is an explanatory diagram schematically showing the correspondence between a manager account and a manager wallet memorized in a blockchain. FIG. 5(B) is an explanatory diagram schematically showing the correspondence between each user account and each user wallet memorized in the blockchain. FIG. 5(C) is an explanatory diagram schematically showing the correspondence between each emitter account and each emitter wallet memorized in the blockchain.
[FIG. 6] FIG. 6 is an explanatory diagram used for describing exchange of data among a management system, a user terminal, an emitter terminal, and respective wallets in the trading system of FIG. 3.
[FIG. 7] FIG. 7 is an explanatory diagram used for describing exchange of data by a method different from the method in FIG. 6.
[FIG. 8] FIG. 8 is an explanatory diagram used for describing a carbon dioxide utilization system to which a carbon dioxide consumption amount calculation system according to a second embodiment is applied, etc.
[FIG. 9] FIG. 9 is an explanatory diagram used for describing a trading system and a support system to which the carbon dioxide consumption amount calculation system shown in FIG. 8 is applied.

### DESCRIPTION OF EMBODIMENTS

Examples of embodiments are listed in the following [1] to [14].
[1] A carbon dioxide consumption amount calculation system comprising:
   a first detection section disposed in a raw material supply passage between a supply section serving as a carbon dioxide supply source and a carbon dioxide utilization device which produces a carbon dioxide-originating product by using, as a raw material, carbon dioxide supplied from the supply section, the first detection section detecting a first parameter related to carbon dioxide supplied via the raw material supply passage;
   a second detection section which detects a second parameter related to carbon dioxide contained in the product produced by the carbon dioxide utilization device; and
   a calculation section which calculates the consumption amount of carbon dioxide consumed in the carbon dioxide utilization device on the basis of the first parameter and the second parameter.
   The carbon dioxide consumption amount calculation system of the above [1] can detect the first parameter related to carbon dioxide supplied via the raw material supply passage by the first detection section, and can detect the second parameter related to carbon dioxide contained in the product, produced by the carbon dioxide utilization device, by the second detection section. The above-described consumption amount calculation system can more accurately calculate "the amount of carbon dioxide consumed in the carbon dioxide utilization device" by reflecting the first parameter obtained at the passage through which carbon dioxide flows before being supplied to the carbon dioxide utilization device and the second parameter based on the product obtained as a result of supply of carbon dioxide to the carbon dioxide utilization device.
[2] The carbon dioxide consumption amount calculation system described in [1], wherein the calculation section calculates the consumption amount on the basis of the difference between the amount of supplied carbon dioxide determined on the basis of the first parameter and the amount of carbon dioxide remaining in the product determined on the basis of the second parameter.
   The difference between "the amount of supplied carbon dioxide determined on the basis of the first parameter" and "the amount of carbon dioxide remaining in the product determined on the basis of the second parameter" is highly likely to coincide with the amount of carbon dioxide consumed in the carbon dioxide utilization device or approximate the amount of carbon dioxide consumed. Therefore, when the carbon dioxide consumption amount calculation system of the above [2] calculates the above-mentioned consumption amount on the basis of the above-mentioned difference, "the amount of carbon dioxide consumed in the carbon dioxide utilization device" can be calculated further accurately.
[3] The carbon dioxide consumption amount calculation system described in [1], wherein the second detection section detects the second parameter related to carbon dioxide contained in the product in a product passage provided on the downstream side of the carbon dioxide utilization device,
   wherein the carbon dioxide consumption amount calculation system further comprises a third detection section which detects a third parameter related to carbon dioxide contained in exhaust gas in an exhaust gas passage provided on the downstream side of the carbon dioxide utilization device such that the exhaust gas other than the product flows through the exhaust gas passage, and
   wherein the calculation section calculates the consumption amount on the basis of the first parameter, the second parameter, and the third parameter.
   In the case where the above-described product and the above-described exhaust gas are produced in the carbon dioxide utilization device, the carbon dioxide consumption amount calculation system of the above [3] can detect the third parameter related to carbon dioxide contained in the exhaust gas by the third detection section. Thus, the above-described consumption amount calculation system can more accurately calculate "the amount of carbon dioxide consumed in the carbon dioxide utilization device" by reflecting the first parameter obtained at the passage through which carbon dioxide flows before being supplied to the carbon dioxide utilization device, the second parameter based on the product obtained as a result of supply of carbon dioxide to the carbon dioxide utilization device, and the third parameter based on carbon dioxide contained in the remaining exhaust gas.
[4] The carbon dioxide consumption amount calculation system described in any one of [1] to [3], comprising a record section which hashes at least one of the first parameter and the second parameter and records the hashed parameter(s) in a blockchain.
   The carbon dioxide consumption amount calculation system of the above [4] can manage at least one of the first parameter and the second parameter by using a blockchain, thereby enhancing the function of preventing unauthorized manipulation of the parameter(s). Therefore, this consumption amount calculation system can operate the system while ensuring the reliability of the parameter(s).
[5] The carbon dioxide consumption amount calculation system described in any one of [1] to [4], wherein the calculation section includes a program for calculating the consumption amount on the basis of the first parameter and the second parameter, and wherein the carbon dioxide consumption amount calculation system further comprises a changing history record section which hashes a changing history of the program and records the hashed changing history in a blockchain.
   The carbon dioxide consumption amount calculation system of the above [5] can manage the program changing history by using the blockchain, thereby enhancing the function of preventing unauthorized manipulation of the program. Therefore, this consumption amount calculation system can operate the system while ensuring the reliability of the program.
[6] The carbon dioxide consumption amount calculation system described in any one of [1] to [5], comprising:
   a data record section which records data in a cloud, the data containing at least one of the first parameter and the second parameter; and
   a comparison section which operates, when at least one of the first detection section and the second detection section generates current data containing at least one of the first parameter and the second parameter, so as to compare the current data with data recorded in the cloud before generation of the current data.
   In the case where the carbon dioxide consumption amount calculation system of the above [6] generates current data containing at least one of the first parameter and the second parameter, the carbon dioxide consumption amount calculation system can compare the current data with the data recorded in the cloud before generation of the current data and verify the current data.
[7] A carbon dioxide consumption amount calculation method comprising:
   calculating the consumption amount of carbon dioxide consumed in a carbon dioxide utilization device on the basis of a first parameter and a second parameter related to carbon dioxide,
   the first parameter being related to carbon dioxide supplied via a raw material supply passage between a supply section serving as a carbon dioxide supply source and the carbon dioxide utilization device which produces a carbon dioxide-originating product by using, as a raw material, carbon dioxide supplied from the supply section, and
   the second parameter being related to carbon dioxide contained in the product produced by the carbon dioxide utilization device.
   The above-described consumption amount calculation method of the above [7] can more accurately calculate "the amount of carbon dioxide consumed in the carbon dioxide utilization device" by reflecting the first parameter related to carbon dioxide supplied via the raw material supply passage and the second parameter related to carbon dioxide contained in the product produced by the carbon dioxide utilization device.
[8] The carbon dioxide consumption amount calculation method described in [7], wherein the consumption amount is calculated on the basis of the difference between the amount of supplied carbon dioxide determined on the basis of the first parameter and the amount of carbon dioxide remaining in the product determined on the basis of the second parameter.
   The difference between "the amount of supplied carbon dioxide determined on the basis of the first parameter" and "the amount of carbon dioxide remaining in the product determined on the basis of the second parameter" is highly likely to coincide with the amount of carbon dioxide consumed in the carbon dioxide utilization device or approximate the amount of carbon dioxide consumed. Therefore, when the above-mentioned consumption amount is calculated by the carbon dioxide consumption amount calculation method of the above [8] on the basis of the above-mentioned difference, "the amount of carbon dioxide consumed in the carbon dioxide utilization device" can be calculated further accurately.
[9] The carbon dioxide consumption amount calculation method described in [7], wherein the consumption amount is calculated on the basis of the first parameter, the second parameter, and a third parameter related to carbon dioxide contained in exhaust gas in an exhaust gas passage provided on the downstream side of the carbon dioxide utilization device such that the exhaust gas other than the product flows through the exhaust gas passage.
   The above-described consumption amount calculation method of the above [9] can more accurately calculate "the amount of carbon dioxide consumed in the carbon dioxide utilization device" by reflecting the first parameter obtained at the passage through which carbon dioxide flows before being supplied to the carbon dioxide utilization device, the second parameter based on the product obtained as a result of supply of carbon dioxide to the carbon dioxide utilization device, and the third parameter based on carbon dioxide contained in the remaining exhaust gas.
[10] A carbon dioxide emission trading support system comprising:
   a management system having a token issuance section for issuing a token; and
   a manager wallet associated with the management system,

   wherein, in the case where a user account and a user wallet are associated with each other, and a consumption signal for notifying an amount of consumption of carbon dioxide is sent from a user terminal associated with the user account to the management system, the token issuance section issues the token on the basis of the consumption signal, and the management system instructs storage of the issued token in the user wallet, and
   wherein the consumption signal is generated and sent on the basis of the consumption amount calculated by the carbon dioxide consumption amount calculation method described in any one of [7] to [9].
   The emission trading support system of the above [10] can provide an incentive to a user contributing to consumption (reduction) of carbon dioxide by sending tokens to the user wallet. Furthermore, in the case where carbon dioxide has been consumed in the utilization device, the above-mentioned emission trading support system can more accurately calculate the amount of consumed carbon dioxide and more properly issue a token(s) on the basis of the calculated consumption amount.
[11] The carbon dioxide emission trading support system described in [10], wherein, when a sell request that requests selling of the token stored in the user wallet is notified from the user terminal to the management system, the management system moves the token from the user wallet to the manager wallet on the basis of the sell request.
   The support system of the above [11] executes a transaction of moving the token from the user wallet to the manager wallet on the basis of the request. By virtue of this, the token stored in the user wallet can be moved to the manager wallet at any time in response to the sell request. Therefore, it becomes easier for the user to use the token more freely.
[12] The carbon dioxide emission trading support system described in [11], wherein, in the case where an emitter account and an emitter wallet are associated with each other, and a buy request that requests buying of the token is notified from an emitter terminal associated with the emitter account to the management system, the management system moves the token from the manager wallet to the emitter wallet on the basis of the buy request.
   In the support system of the above [12], in the case where a sell request is sent from the user terminal and a buy request is sent from the emitter terminal, on the basis of the requests, the management system executes a transaction of moving the token from the manger wallet to the emitter wallet. By virtue of this, it is possible to move the token from the user wallet to the manager wallet once and then moves the token to the emitter wallet. This support system easily moves the token from the user wallet to the emitter wallet even when the timing of the sell request and the timing of the buy request differ.
[13] The support system for carbon dioxide emission trading described in [10] or
   , wherein, in the case where an emitter account and an emitter wallet are associated with each other, and a buy request that requests buying of the token is notified from an emitter terminal associated with the emitter account to the management system and a sell request that requests selling of the token stored in the user wallet is notified from the user terminal to the management system, the management system moves the token from the user wallet to the emitter wallet on the basis of the buy request and the sell request.
   In the support system of the above [13], in the case where a sell request is sent from the user terminal and a buy request is sent from the emitter terminal, on the basis of the requests, the management system executes a transaction of moving the token from the user wallet to the emitter wallet. By virtue of this, it is possible to move the token from the user wallet to the emitter wallet while eliminating temporary storage of the token in the manger wallet. Therefore, the support system can further reduce the burden of the manager wallet.
[14] A carbon dioxide emission trading support method which uses:
   a management system having a token issuance section for issuing a token, and
   a manager wallet associated with the management system,

   wherein, in the case where a user account and a user wallet are associated with each other, and a consumption signal for notifying an amount of consumption of carbon dioxide is sent from a user terminal associated with the user account to the management system, the token issuance section issues the token on the basis of the consumption signal, and the management system instructs storage of the issued token in the user wallet, and
   wherein the consumption signal is generated and sent on the basis of the consumption amount calculated by the carbon dioxide consumption amount calculation method described in any one of [7] to [9].

### <First embodiment >

### 1. Outline of carbon dioxide utilization system 9

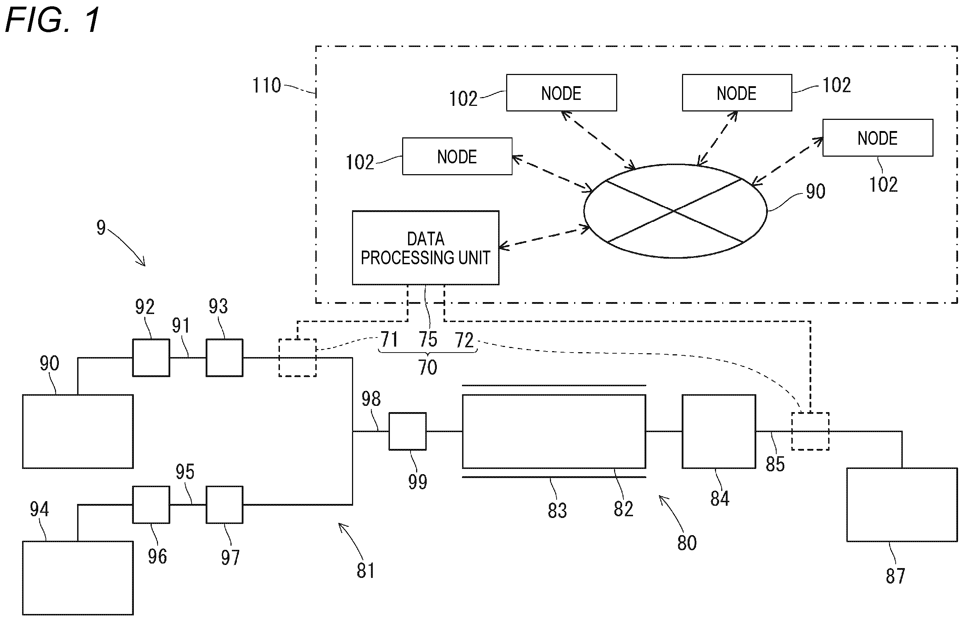

A carbon dioxide utilization system 9, etc. are shown in FIG. 1. The carbon dioxide utilization system 9 will be also referred to simply as the utilization system 9. The utilization system 9 mainly includes a raw material feed section 81, a carbon dioxide utilization device 80, and a carbon dioxide consumption amount calculation system 70. The carbon dioxide utilization device 80 will be also referred to simply as the utilization device 80. The carbon dioxide consumption amount calculation system 70 will be also referred to simply as the consumption amount calculation system 70. The utilization system 9 is a system which can produce a carbon dioxide-originating product by consuming carbon dioxide fed as a raw material and which can calculate the amount of carbon dioxide consumed in the production process.

The raw material feed section 81 includes a carbon dioxide supply section 90, a first supply passage 91, a control valve 92, a check valve 93, a hydrogen supply section 94, a second supply passage 95, a control valve 96, a check valve 97, a merging passage 98, and a gate valve 99. The raw material feed section 81 is a section for feeding a plurality of types of materials used for producing a carbon dioxide-originating product (for example, methane).

The carbon dioxide supply section 90 corresponds to one example of the supply section. The carbon dioxide supply section 90 functions as a source for supplying carbon dioxide. The carbon dioxide supply section 90 is, for example, a tank which can store and keep carbon dioxide in a liquid state. The carbon dioxide in the carbon dioxide supply section 90 is, for example, carbon dioxide generated in a carbon dioxide generating location (for example, a plant or the like), recovered by a known method, and liquefied. However, no limitation is imposed on the method for preparing carbon dioxide which is fed into the carbon dioxide supply section 90, and various known methods may be employed. The first supply passage 91 corresponds to one example of the raw material supply passage and functions as a flow passage which allows flow of carbon dioxide between the carbon dioxide supply section 90 (the supply section) and the utilization device 80. The first supply passage 91 is a passage for supplying carbon dioxide from the carbon dioxide supply section 90 to the utilization device 80. The first supply passage 91 is piping through which carbon dioxide (first raw material gas) supplied from the carbon dioxide supply section 90 flows, and the control valve 92 and the check valve 93 are disposed in this order from the upstream side toward the downstream side.

The hydrogen supply section 94 is a tank which can store and keep hydrogen produced by a known hydrogen production system (for example, a system for producing hydrogen by using a solid oxide electrolysis cell (SOEC)). The second supply passage 95 is piping through which hydrogen (second raw material gas) supplied from the hydrogen supply section 94 flows, and the control valve 96 and the check valve 97 are disposed in this order from the upstream side toward the downstream side. The merging passage 98 is merging piping which is connected to the downstream end of the first supply passage 91 and the downstream end of the second supply passage 95. The merging passage 98 merges carbon dioxide flowing thereinto through the first supply passage 91 and hydrogen flowing thereinto through the second supply passage 95 and feeds the mixture of carbon dioxide and hydrogen to the reaction vessel 82. The gate valve 99 is disposed in the merging passage 98. Carbon dioxide (the first raw material gas) and hydrogen (the second raw material gas) flow through the control valves 92 and 96, respectively, whereby carbon dioxide and hydrogen are mixed at an optimal mixing ratio. The gas mixture (raw material gas) of the two raw material gases is supplied to the reaction vessel 82 via the gate valve 99. In the representative example, the mixing ratio is set such that the carbon dioxide supplied from the carbon dioxide supply section 90 and hydrogen supplied from the hydrogen supply section 94 have a molar ratio of 1 : 4, and carbon dioxide and hydrogen are supplied to the reaction vessel 82 at this molar ratio.

The utilization device 80 is a device which produces a carbon dioxide-originating product by using, as a raw material, carbon dioxide supplied from the carbon dioxide supply section 90. The utilization device 80 includes a reaction vessel 82, a heater 83, a condenser 84, a downstream flow passage 85, a tank 87, etc. In the representative example described below, the utilization device 80 is configured as a methanation device which causes the plurality of types of raw materials (raw materials containing carbon dioxide) fed by the raw material feed section 81 to react with each other, thereby generating methane as a carbon dioxide-originating product.

The reaction vessel 82 is a vessel through which the gas mixture flowing thereinto through the merging passage 98 flows and in which a reaction occurs. The reaction vessel 82 has an appropriately set pressure and is heated by the heater 83, whereby the Sabatier reaction represented by the following chemical reaction formula is conducted, and thus, production of methane (methanation) is performed.

CO₂ + 4H₂ → CH₄ + 2H₂O

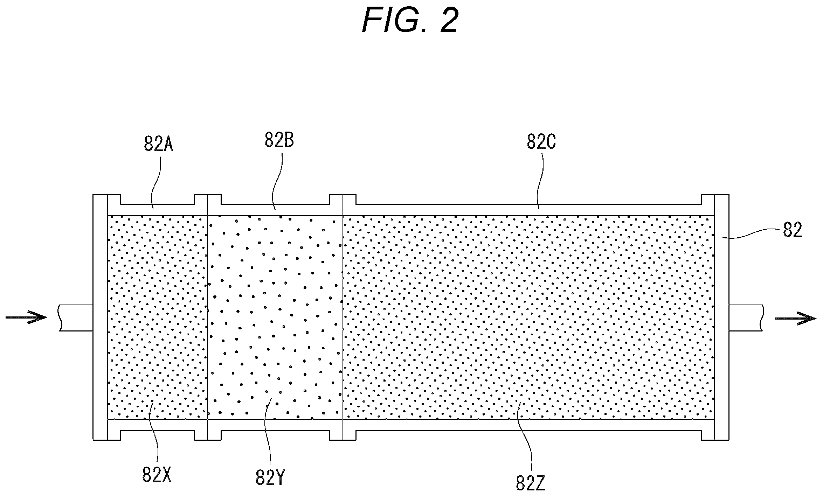

FIG. 2 is a sectional view showing, as an example, the internal structure of the reaction vessel 82. The arrows shown in FIG. 2 indicate the direction in which the raw material gas flows through the reaction vessel 82. The reaction vessel 82 contains catalysts 82X and 82Z and a low-activity catalyst 82Y. The reaction vessel 82 includes a first section 82A, a second section 82B, and a third section 82C arranged in this order along the flow direction of the raw material gas (from the upstream side toward the downstream side) and is configured such that the gas flows through the first section 82A, the second section 82B, and the third section 82C in this order. When the raw material gas, which is the mixture of hydrogen and carbon dioxide is fed from the merging passage 98 into the reaction vessel 82, the raw material gas flows through the first section 82A, the second section 82B, and the third section 82C in this order, and, in the reaction vessel 82, a chemical reaction forming methane and water proceeds.

In the representative example, the first section 82A and the third section 82C accommodate the catalysts 82X and 82Z, respectively. The second section 82B accommodates the low-activity catalyst 82Y having a catalytic activity lower than that of the catalyst 82X or 82Z. The catalysts 82X and 82Z and the low-activity catalyst 82Y lower the activation energy of chemical reaction, to thereby facilitate progress of the chemical reaction. A low catalytic activity refers to a small degree of lowering the activation energy of chemical reaction. Moreover, in the representative example, the catalytic activity of the catalyst 82X in the first section 82A is equal to that of the catalyst 82Z in the third section 82C, or lower than that of the catalyst 82Z in the third section 82C. In addition, in the representative example, the thickness of the first section 82A in the raw material gas flow direction is smaller than that of the third section 82C in the raw material gas flow direction. However, the present invention is not limited to such an representative example, and the specific internal configuration of the reaction vessel 82 can be changed in various ways so long as the reaction vessel 82 has a configuration which enables production of methane (methanation) represented by the chemical reaction formula: CO₂ + 4H₂ → CH₄ + 2H₂O. For example, the types of the catalysts, the thicknesses of the first section 82A, the second section 82B, and the third section 82C are not limited to those in the above-described example and can be changed variously. Also, in the representative example, the first section 82A, the second section 82B, and the third section 82C are provided, whereby the interior of the reaction vessel 82 is divided to three regions. However, the interior of the reaction vessel 82 may be divided to four or more regions, and the number of the regions may be two or less.

No particular limitation is imposed on the catalysts 82X and 82Z and the low-activity catalyst 82Y, so long as the catalysts are adapted to various chemical reaction. Examples of the catalysts 82X and 82Z and the low-activity catalyst 82Y include powder, pellets, and a porous body of a particle-on-carrier. Examples of the carrier include powder, pellets, and a porous body of an oxide including one or more species of alumina, silica, magnesia, titania, zirconia, niobia, silica-alumina, zeolite, and calcium phosphate. The porous body has gas permeability which allows the raw material gas to pass through. In the case of powder or pellets, the raw material gas passes through voids therein.

Examples of the material of the particles supported on the carrier include metals including one or more elements of Fe, Co, Ni, Cu, Ru, Rh, Pd, Ag, Ir, Pt, and Au. Regarding the catalyst, if the same material and particle size of the carrier and particles are employed, catalytic activity increases in proportion to the surface area of the particles supported on the carrier. Thus, by reducing the surface area of the particles supported on the carrier with respect to that of the catalysts 82X and 82Z, the low-activity catalyst 82Y can be provided.

Also, when inert particles having no catalytic activity are added to the catalyst 82X or 82Z so as to reduce the relative amount of catalyst 82X or 82Z in a specific amount, the low-activity catalyst 82Y is provided. Examples of the inert particle species include powder and pellets of an oxide including one or more species of alumina, silica, magnesia, titania, zirconia, niobia, silica-alumina, zeolite, and calcium phosphate.

The product produced as a result of the reaction in the reaction vessel 82 is fed to the condenser 84 connected to the downstream end of the reaction vessel 82 and is cooled by the condenser 84, whereby the product is separated to water and gas containing methane. "the gas containing methane" separated by the condenser 84 may contain hydrogen and carbon dioxide of the raw material gas or may not contain one or both of them. In the representative example of the first embodiment, the mixing ratio is set in the raw material feed section 81 such that the molar ratio of carbon dioxide and hydrogen becomes 1 : 4, and carbon dioxide and hydrogen are supplied to the reaction vessel 82 at this molar ratio. Namely, in the representative example, since carbon dioxide is completely consumed in the reaction process, "the gas containing methane" separated by the condenser 84 is methane containing no carbon dioxide. This methane flows through the downstream flow passage 85 as a carbon dioxide-originating product. The methane having flowed through the downstream flow passage 85 is compressed by, for example, an unillustrated compressor and is stored in the tank 87. The tank 87 is, for example, a methane cylinder in which compressed methane is stored.

### 2. Details of carbon dioxide consumption amount calculation system 70

The consumption amount calculation system 70 is a system for calculating the amount of carbon dioxide consumed by the utilization device 80. The consumption amount calculation system 70 has not only the function of calculating the carbon dioxide consumption amount but also various other functions. The consumption amount calculation system 70 mainly includes a first detection section 71, a second detection section 72, and a data processing unit 75.

The first detection section 71 is a device which detects a first parameter related to carbon dioxide supplied via the first supply passage 91. In the representative example, the first detection section 71 detects the flow rate of CO₂ (carbon dioxide) flowing through the first supply passage 91. The first detection section 71 includes, for example, a flowmeter and a gas chromatography. The flowmeter measures the flow rate of the gas flowing through the first supply passage 91. The gas chromatography measures the concentrations (volume ratios) of components (CO₂, H₂O, etc.) contained in the gas flowing through the first supply passage 91. For example, in the case where the flow rate of the gas flowing through the first supply passage 91, which is measured by the flowmeter contained in the first detection section 71, is 100 L/min and the result of measurement by the gas chromatography contained in the first detection section 71 shows that, in the gas flowing through the first supply passage 91, the volume ratio of CO₂ is 20 vol% and the volume ratio of H₂O is 0 vol%, it is determined that the volume ratio of H₂ is 80 vol%. Therefore, it is possible to determine, on the basis of the above-mentioned flow rate and the volumes ratios of the respective gases, that the flow rate of CO₂ flowing through the first supply passage 91 is 20 L/min, the flow rate of H₂ flowing through the first supply passage 91 is 80 L/min, and the flow rate of H₂O flowing through the first supply passage 91 is 0 L/min. In this example, the flow rate of CO₂ flowing through the first supply passage 91 corresponds to one example of the first parameter.

The second detection section 72 is a device which detects a second parameter related to carbon dioxide contained in the product produced by the carbon dioxide utilization device 80. In the representative example, the second detection section 72 detects the flow rate of methane flowing through the downstream flow passage 85. The second detection section 72 includes, for example, a flowmeter and a gas chromatography. The flowmeter measures the flow rate of the gas in the downstream flow passage through which the product produced by the utilization device 80 flows. The gas chromatography measures the concentrations (volume ratios) of components contained in the gas flowing through the downstream flow passage. For example, in the case where the flow rate of the gas flowing through the downstream flow passage 85, which is measured by the flowmeter contained in the second detection section 72, is 60 L/min and the result of measurement by the gas chromatography contained in the second detection section 72 shows that, in the gas flowing through the downstream flow passage 85, the volume ratio of CO₂ is 0 vol%, the volume ratio of H₂O is 67 vol%, the volume ratio of CH₄ is 33 vol%, and the volume ratio of CO is 0 vol%, it is possible to determine that the flow rate of CO₂ flowing through the downstream flow passage 85 is 0 L/min, the flow rate of H₂O flowing though the downstream flow passage 85 is 40 L/min, the flow rate of CH₄ flowing though the downstream flow passage 85 is 20 L/min, and the flow rate of CO flowing though the downstream flow passage 85 is 0 L/min. In this example, the flow rate of CO₂ flowing though the downstream flow passage 85 corresponds to one example of the second parameter.

The data processing unit 75 corresponds to one example of the calculation section. The data processing unit 75 calculates the amount of carbon dioxide consumed in the utilization device 80 on the basis of the first parameter and the second parameter mentioned above. For example, the data processing unit 75 (the calculation section) can calculate the consumption amount in the utilization device 80 on the basis of the difference between the carbon dioxide supply amount determined on the basis of the first parameter and the amount of carbon dioxide remaining in the product determined on the basis of the second parameter. "The consumption amount in the utilization device 80" to be calculated may be the consumption amount per unit time or the total consumption amount during a predetermined period for which calculation is performed. For example, the difference (X1 - X2) between the flow rate X1 of CO₂ flowing through the first supply passage 91 and the flow rate X2 of CO₂ flowing through the downstream flow passage 85 may be calculated as the carbon dioxide consumption amount (specifically, consumption amount per unit time). For example, in the above-described specific example, the flow rate X1 of CO₂ flowing through the first supply passage 91 is 20 L/min and the flow rate X2 of CO₂ flowing through the downstream flow passage 85 is 0 L/min, and therefore, 20 L/min, which is the difference (X1 - X2) between the flow rate X1 and the flow rate X2, may be used as the carbon dioxide consumption amount in the utilization device 80.

The data processing unit 75 (the calculation section) includes a program for calculating the consumption amount on the basis of the first parameter and the second parameter. The program includes an expression or table for calculating the consumption amount using the first parameter and the second parameter as variables.

The data processing unit 75 functions as a record section which records data, containing at least one of the first parameter and the second parameter, in a cloud. The cloud may be a cloud system including a blockchain 110, which will be described later, or a storage system (storage system including a server or the like) which is not a blockchain. Although the data containing at least one of the first parameter and the second parameter may be measurement data, the data may be data obtained by converting the measurement data by hashing or data obtained by hashing the measurement data together with serial numbers corresponding to the detection sections. In the representative example described below, the case where the data containing the first parameter and the second parameter are hashed and the hashed data are stored in the blockchain 110 will be described as an example.

The data processing unit 75 functions as a record section which records either of the first parameter and the second parameter in the blockchain after hashing it. In the present embodiment, the utilization system 9 includes a plurality of nodes 102. Each node 102 is a device for implementing the blockchain. The blockchain implemented by the plurality of nodes 102 may be a public blockchain (public chain), a private blockchain (private chain), or any other type of blockchain.

In the case where the utilization system 9 is implemented as a private blockchain, when data are collected by the data processing unit 75, a transaction indicating the data contents is generated, and the generated transaction is verified and approved by the plurality of nodes 102. Then, a predetermined node such as a manager node (for example, a node 102 constituting the utilization system 9) or a node designated by the manager node or the like calculates a hash value, and a block is configured from the approved transaction by using the calculated hash value. The configured block is added to the blockchain and is distributed to and stored in each node 102. Meanwhile, in the case where the utilization system 9 is implemented as a public blockchain, when the data are collected, a transaction indicating the details of the data collection is generated, and the generated transaction is verified and approved by a node 102. Then, one of miners calculates a hash value, and a block is configured from the approved transaction by using the calculated hash value. The configured block is added to the blockchain and is distributed to and stored in each node 102.

The data processing unit 75 also functions as a record section which hashes the changing history of the above-described program (the program for calculating the consumption amount on the basis of the first parameter and the second parameter) and records the hashed changing history in the blockchain.

The data processing unit 75 or a management device different from the data processing unit 75 may function as a comparison section. In this case, when at least one of the first detection section 71 and the second detection section 72 generates current data containing at least one of the first parameter and the second parameter, the comparison section can compare the current data with the data recorded in the cloud before generation of the current data and verify the current data. In this example, it is desired that the current data and the data recorded in the cloud are, for example, hashed data. Moreover, data based on the serial values obtained in the first detection section 71 and the second detection section 72 may be compared. The comparison of data may be performed at a predetermined interval (e.g., every 1 hour) by the data processing unit 75, or a management device which stores the data recorded in the cloud, or the like.

### 3. Configuration of carbon dioxide emission trading system 1

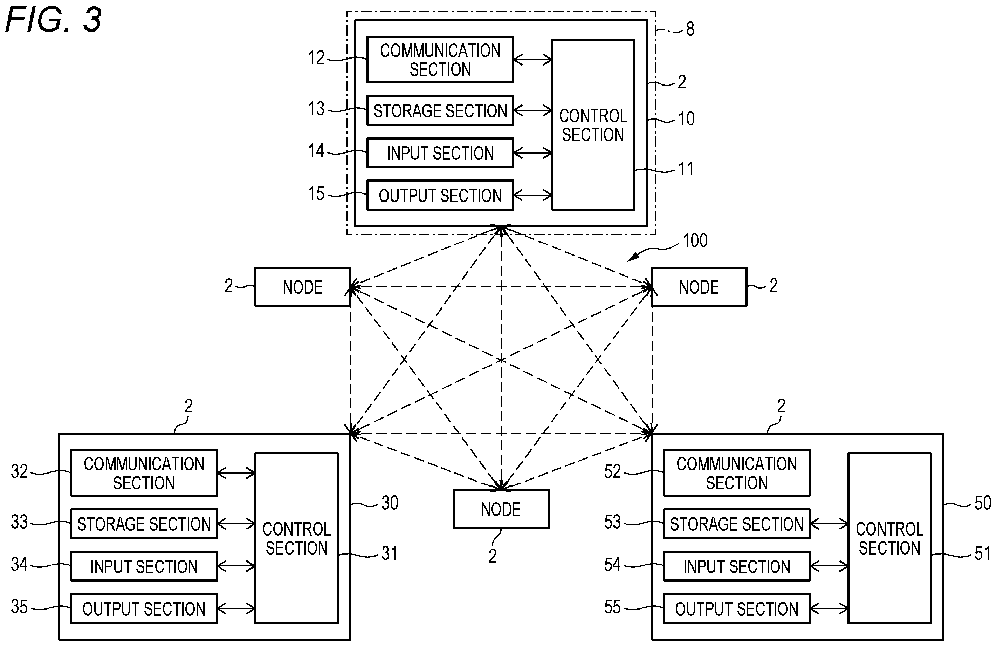

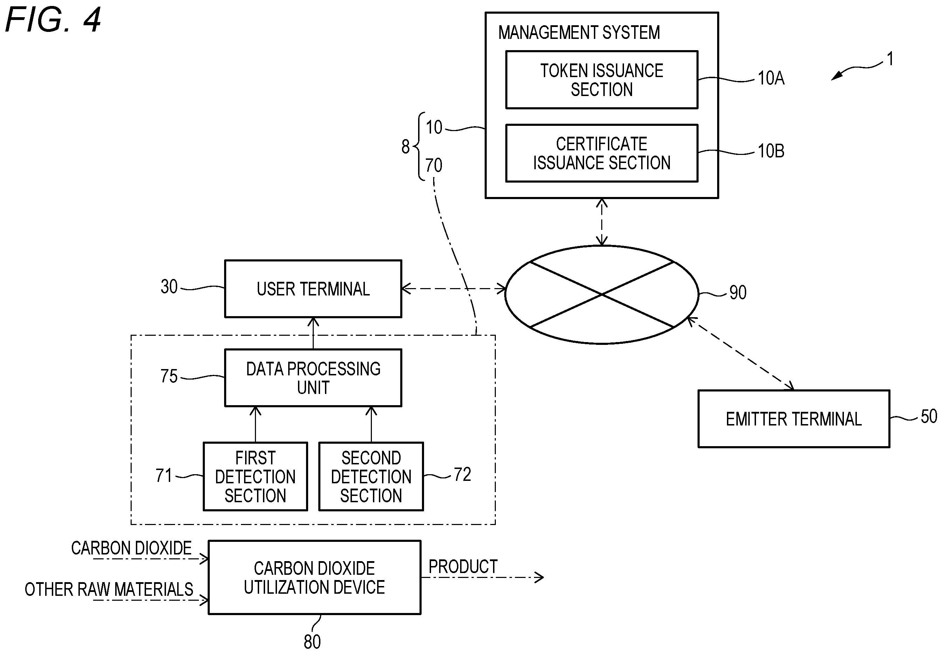

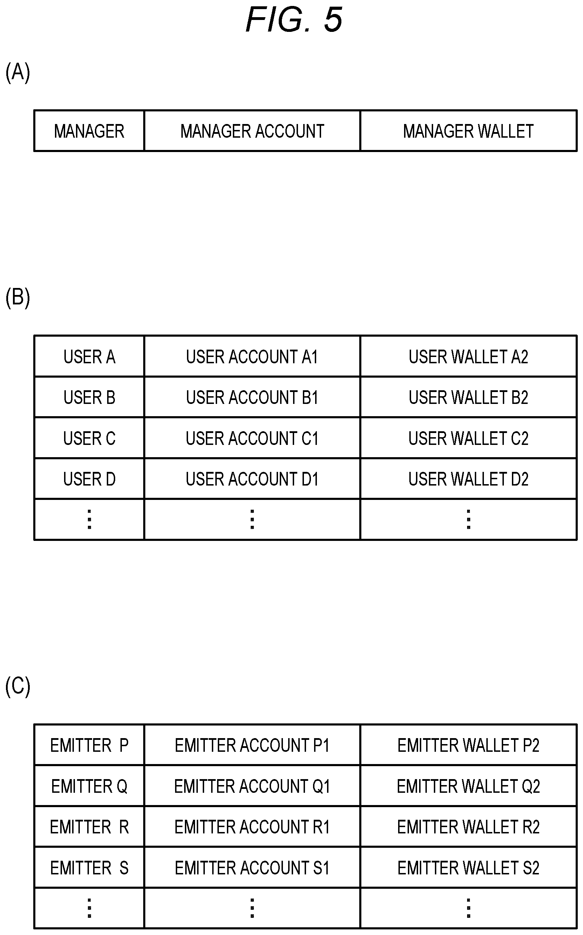

FIG. 3 is an explanatory diagram schematically showing a carbon dioxide emission trading system 1. The carbon dioxide emission trading system 1 will be also referred to simply as the trading system 1. In the trading system 1 of the representative example, which will be described below, at least the above-described consumption amount calculation system 70, a management system 10, and a manager wallet 20 (FIG. 5) are provided so as to realize a carbon dioxide emission trading support system 8. Notably, in FIGS. 3 and 4, the manager wallet 20 (FIG. 5) is not shown. In FIG. 3, the consumption amount calculation system 70 is not shown. The carbon dioxide emission trading support system 8 will be also referred to simply as the support system 8.

The trading system 1 has a plurality of nodes 2. Each node 2 is a device for implementing a blockchain. The blockchain realized by the plurality of nodes 2 may be a public blockchain (public chain), a private blockchain (private chain), or any other type of blockchain. Notably, the blockchain 100 shown in FIG. 3 and the blockchain 110 shown in FIG. 1 may be different blockchains or may be a common blockchain.

In the case where the trading system 1 is implemented as a private blockchain, when each trade described below is executed, a transaction indicating the details of the trade is generated, and the generated transaction is verified and approved by the plurality of nodes 2. Then, a predetermined node such as a manager node (for example, a node 2 constituting a management system 10) or a node designated by the manager node or the like calculates a hash value, and a block is configured from the approved transaction by using the calculated hash value. The configured block is added to the blockchain and is distributed to and stored in each node 2. Meanwhile, in the case where the trading system 1 is implemented as a public blockchain, when each trade described below is executed, a transaction indicating the details of the trade is generated, and the generated transaction is verified and approved by a node 2. Then, one of miners calculates a hash value, and a block is configured from the approved transaction by using the calculated hash value. The configured block is added to the blockchain and is distributed to and stored in each node 2.

Each of the nodes 2 constituting the trading system 1 is configured by an information processing device, for example, a server, a personal computer, or the like. Although six nodes 2 are shown in FIG. 3 as an example, the number of the nodes 2 may be larger or smaller than that in the example of FIG. 3.

Some of the nodes 2 constitute the management system 10. The management system 10 includes a control section 11, a communication section 12, a storage section 13, an input section 14, an output section 15, etc. The storage section 13 is a device for storing programs, parameters, etc. therein. The control section 11 executes various functions and processes of the node 2 in accordance with the programs and various types of data (parameters, etc.) stored in the storage section 13. The communication section 12 is a device for performing wired communication or wireless communication with an external device in cooperation with the control section 11. The input section 14 is an input device such as an operation device, and the output section 15 is an output device such as a display device. The management system 10 may be composed of one device (e.g., one server device) or may be composed of a plurality of devices.

Some of the nodes 2 constitute a user terminal 30. The user terminal 30 includes a control section 31, a communication section 32, a storage section 33, an input section 34, an output section 35, etc. The hardware configuration of the user terminal 30 may be the same as or different from the hardware configuration of the management system 10.

Some of the nodes 2 constitute an emitter terminal 50. The emitter terminal 50 includes a control section 51, a communication section 52, a storage section 53, an input section 54, an output section 55, etc. The hardware configuration of the emitter terminal 50 may be the same as or different from the hardware configuration of the management system 10.

In FIG. 3, the specific configurations of the nodes 2 other than the management system 10, the user terminal 30, and the emitter terminal 50 are not shown. However, any of the nodes 2 whose configurations are not shown may be configured as the user terminal 30 or may be configured as the emitter terminal 50. The user terminal 30 shown in FIG. 3 may have the function of the emitter terminal 50. The emitter terminal 50 may have the function of the user terminal 30.

As shown in FIG. 4, a token issuance section 10A and a certificate issuance section 10B are contained in the management system 10. In the representative example which will be described below, the control section 11 (FIG. 3) functions as the token issuance section 10A and the certificate issuance section 10B. The token issuance section 10A has a function of issuing a token. The certificate issuance section 10B has a function of issuing an emission right use certificate and a consumption certificate. The token is a virtual currency or right used for trading in the trading system 1.

As shown in FIG. 4, at a site where the user terminal 30 is disposed, the above-described utilization device 80 and the above-described consumption amount calculation system 70 are provided.

In the present embodiment, as shown in FIG. 5(A), a manager account is assigned to a manager such that the manager account is associated with the manager, a manager wallet 20 is associated with the manager account, and the manager account and the manager wallet are stored in the blockchain 100 such that they are associated with each other. The manager account is a piece of identification information for specifying the manager. The manager is an entity which manages the issuance of tokens. In the blockchain 100, when the manager account is specified, the content of the manager wallet 20 is specified. The manager wallet 20 is a wallet associated with the management system 10. The manager wallet 20 is a virtual place where tokens handled by the management system 10 (manager's tokens) are stored and is a virtual place associated with the manager account.

In the present embodiment, user accounts are assigned to respective users such that the user accounts are associated with the respective users, and user wallets 40 are associated with the user accounts. For example, as shown in FIG. 5(B), the respective user accounts and the respective user wallets are stored in the blockchain 100 such that the respective user wallets are associated with the respective user accounts. Each user account is an account associated with a corresponding user wallet and a piece of identification information for specifying a user. The user is an entity which receives an issued token(s) by consuming carbon dioxide. The user wallet is a virtual place where tokens owned by the user are stored and is a virtual storage place associated with the user account. In the blockchain 100, for example, when a user account A1 is specified, the content of a user wallet A2 associated with the user account A1 is found. The user terminal 30 is a terminal used when communications and/or trading based on the user account is performed and is a terminal associated with the user account. Ther user terminal 30 may include the consumption amount calculation system 70 (FIG. 4) or may be separated from the consumption amount calculation system 70.

In the present embodiment, emitter accounts are assigned to respective emitters such that the emitter accounts are associated with the respective emitters, and emitter wallets 60 are associated with the emitter accounts. For example, as shown in FIG. 5(C), the respective emitter accounts and the respective emitter wallets are stored in the blockchain 100 such that the respective emitter wallets are associated with the respective emitter accounts. Each emitter account is an account associated with a corresponding emitter wallet and a piece of identification information for specifying an emitter. The emitter is an entity which buys tokens. Notably, any user may become an emitter, and any emitter may become a user. The emitter wallet is a virtual place where tokens owned by the emitter are stored and is a virtual storage place associated with the emitter account. In the blockchain 100, for example, when an emitter account P1 is specified, the content of an emitter wallet P2 associated with the emitter account P1 is found. The emitter terminal 50 (FIG. 3, etc.) associated with the emitter account is a terminal used when communications and/or trading based on the emitter account is performed.

### 4. Operation of carbon dioxide emission trading system 1

In the trading system 1, the user terminal 30 has a function of generating and sending a consumption signal on the basis of the result of monitoring by the consumption amount calculation system 70. Specifically, the user terminal 30 generates and sends the consumption signal on the basis of the consumption amount calculated by the consumption amount calculation method performed by the consumption amount calculation system 70.

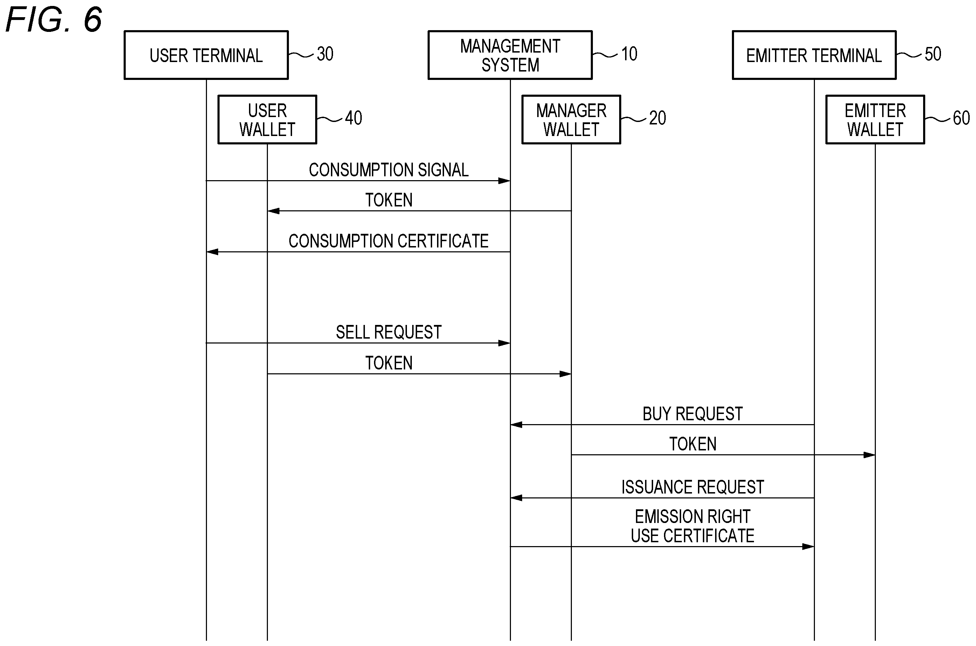

**In** the case where carbon dioxide has been consumed in the carbon dioxide utilization device 80, the consumption amount calculation system 70 detects the amount of carbon dioxide consumed by the carbon dioxide utilization device 80 and notifies the detected carbon dioxide consumption amount to the user terminal 30. **In** the case where the user terminal 30 has obtained the carbon dioxide consumption amount from the consumption amount calculation system 70, as shown in FIG. 6, the user terminal 30 sends to the management system 10 a consumption signal containing a piece of information representing the consumption amount. Since the consumption amount calculation system 70 continuously monitors the amount of carbon dioxide consumed in the utilization device 80 per unit time (L/min), it is possible to calculate the amount (volume or weight) of carbon dioxide consumed in the utilization device 80 at a constant time interval (e.g., every few hours, every day, every few days, etc.). In such an example, the user terminal 30 may be configured such that, every time the consumption amount calculation system 70 calculates "the amount of carbon dioxide consumed in the utilization device 80 at the constant time interval," the user terminal 30 obtains the calculated value (the consumption amount) and sends a consumption signal, containing the obtained calculated value (the consumption amount), to the management system 10 every time the user terminal 30 obtains the calculated value. Notably, the calculation performed period for which the consumption amount calculation system 70 calculates the consumption amount in the utilization device 80 is not limited to periods at the constant time interval, and may be any period of time.

As shown in FIG. 6, in the case where the consumption signal (a signal which notifies the amount of carbon dioxide consumed by the user) is sent to the management system 10 from the user terminal 30 associated with the user account, the token issuance section 10A (FIG. 4) of the management system 10 issues a token(s) on the basis of the consumption signal. Since a consumption amount of carbon dioxide per token is determined beforehand, on the basis of this information, the token issuance section 10A converts to tokens the consumption amount specified by the consumption signal sent by the user terminal 30. The consumption amount of carbon dioxide per token is, for example, 1 ton. In this example, in the case where the consumption amount of carbon dioxide specified by the consumption signal is 10 tons, the token issuance section 10A issues 10 tokens. When the token issuance section 10A issues a token(s), the token issuance section 10A determines the validity period of the token(s). The validity period may be, for example, a period from the date of issue until a predetermined number of days elapses (e.g., until 8 years elapses), or until a predetermined date comes.

The token issuance section 10A also functions as an invalidation section and performs a process of invalidating the token(s) when the validity period set for the token(s) has elapsed. In the case where the token issuance section 10A invalidates a token(s), the token issuance section 10A may instruct a wallet which possesses the token(s) whose validity period has elapsed to delete the token(s), or may invalidate the token(s) by any of other methods.

In the case where the token issuance section 10A has issued a token(s), the management system 10 (for example, the token issuance section 10A) instructs storage of the issued token(s) in the user wallet 40. In the case where the token issuance section 10A has issued a token(s), the management system 10 (for example, the token issuance section 10A) may instruct storing the issued token(s) in the user wallet 40 directly or instruct storing the token(s) in the manager wallet 20 once and moving the token(s) to the user wallet 40 so as to store the token(s) therein. In either case, the issued token(s) is finally stored in the user wallet 40.

In the case where, in response to the consumption signal sent from the user terminal 30 to the management system 10, the token issuance section 10A has issued a token(s) to the user wallet 40, the certificate issuance section 10B of the management system 10 issues a consumption certificate to the user account on the basis of the above-mentioned consumption signal. The consumption certificate includes a piece of information which certifies that carbon dioxide has been consumed in the consumption amount specified by the above-mentioned consumption signal. The consumption certificate includes, as pieces of information, the name of the user specified by the user account and the fact that carbon dioxide has been consumed in the above-mentioned consumption amount. In addition, a piece of information related to a certifier (for example, the name of the manager) may be included. The above-mentioned consumption certificate is desirably electronic data containing symbols, such as letters and numerals, and images. In the case of the consumption certificate is the above-mentioned electronic data, the user terminal 30 which can perform communications and trading on the basis of the user account can obtain the above-mentioned consumption certificate. In the case where the above-mentioned consumption certificate is issued (given) to the user account, the user terminal 30 can store and use the consumption certificate as electronic data. Alternatively, the above-mentioned consumption certificate may be formed on a medium, such as a sheet of paper, by means of, for example, printing.

In the present embodiment, the management system 10 records at least one (preferably, both) of the history of consumption signals and/or the history of trading of tokens in the above-mentioned blockchain after hashing them. Accordingly, the history of all the consumption signals sent to the management system 10 and all the histories of trading of tokens performed between the plurality of nodes 2 are hashed and then recorded in the blockchain.

As shown in FIG. 6, in the case where a sell request that requests selling of a token(s) stored in the user wallet 40 is notified from the user terminal 30 to the management system 10, the management system 10 moves the token(s) from the user wallet 40 to the manager wallet 20 on the basis of the sell request.

In the case where a buy request that requests buying of a token(s) is notified from the emitter terminal 50 associated with the emitter account to the management system 10, the management system 10 moves the token(s) from the manager wallet 20 to the emitter wallet 60 on the basis of the buy request. Notably, the buy request sent from the emitter terminal 50 to the management system 10 may be a buy request specifying only the amount of tokens, or a buy request specifying the amount of tokens and a shortest validity period.

As shown in FIG. 6, in the case where a token(s) is stored in the emitter wallet 60, after an issuance request that requests issuance of an emission right use certificate is notified from the emitter terminal 50 to the management system 10, the management system 10 deletes the token(s) stored in the emitter wallet 60 on the basis of the above-mentioned issuance request, and the certificate issuance section 10B issues the emission right use certificate to the emitter account. By virtue of this, the emitter can use a token(s) (for example, a purchased token(s) ) and receives an emission right use certificate as a certificate that certifies use of the emission right. Notably, the above-mentioned issuance request can make a request while notifying, as an upper limit, the amount of tokens stored in the wallet, or can make a request so as to use only a portion of the tokens stored in the wallet. For example, in the case where 10 tokens are stored in the emitter wallet 60, the emitter terminal 50 can receive a certificate that certifies use of any amount of emission rights by notifying an issuance request while specifying the amounts of tokens in the range of 1 to 10 tokens. The above-mentioned emission right use certificate is a certificate that certifies that the token(s) has been used. Specifically, of the tokens stored (owned) in the emitter wallet 60, tokens for which issuance of an emission right use certificate is requested by the above-mentioned issuance request are deleted by the management system 10, and an emission right use certificate containing a piece of information specifying that tokens the amount of which is equal to the amount of deleted tokens have been used is given to the emitter account. In the emission right use certificate, for example, the fact that the amount of tokens requested by the above-mentioned issuance request have been used is contained as a piece of information. In addition, the name of the emitter specified by the above-mentioned emitter account, and a piece of information related to the certifier (for example, the name of the manager) may be contained. The above-mentioned emission right use certificate is desirably electronic data containing symbols, such as letters and numerals, and images. In the case where the emission right use certificate is the above-mentioned electronic data, the emitter terminal 50 which can perform communications and trading on the basis of the emitter account can obtain the above-mentioned emission right use certificate. In the case where the above-mentioned emission right use certificate is issued (given) to the emitter account, the emitter terminal 50 can store and use the emission right use certificate as electronic data. Alternatively, the above-mentioned emission right use certificate may be formed on a medium, such as a sheet of paper, by means of, for example, printing. Notably, in the present specification, "a predetermined amount of tokens" function as an emission right for emitting carbon dioxide in an amount corresponding to the predetermined amount of tokens. "Use of the tokens stored (owned) in the emitter wallet 60" means use of emission rights in an amount corresponding to the amount of tokens which are used (tokens which are deleted). Accordingly, the emission right use certificate may contain a piece of information that specifies emission rights to be used (for example, a piece of information that certifies the right (emission right) of emitting carbon dioxide in an amount corresponding to the amount of tokens which are used (tokens which are deleted)).

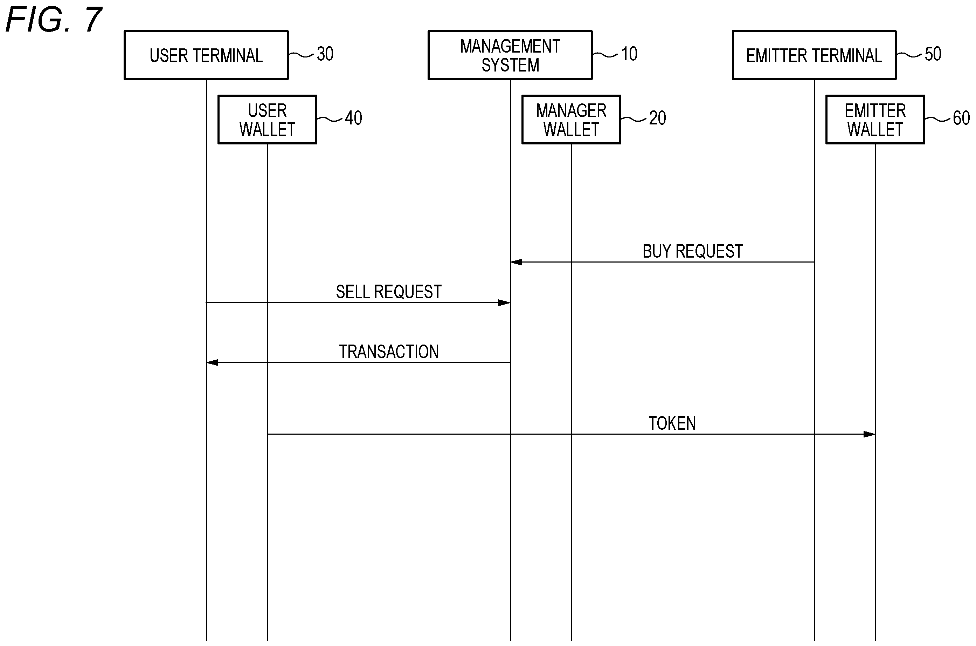

Notably, the trading shown in FIG. 6 may be changed as shown in FIG. 7. In the example of FIG. 7, in the case where a buy request that requests buying of a token(s) is notified from the emitter terminal 50 to the management system 10 and a sell request that requests selling of a token(s) stored in the user wallet 40 is notified from the user terminal 30 to the management system 10, on the basis of the buy request and the sell request, the management system 10 executes a transaction of moving a token(s) from the user wallet 40 to the emitter wallet 60. The above-mentioned transaction is an instruction of moving a token(s) from the user wallet 40 directly to the emitter wallet 60, and the user terminal 30 having received this transaction performs a process of moving a token(s) from the user wallet 40 directly to the emitter wallet 60. In this example, in the case where a sell request is sent from the user terminal 30 and a buy request is sent from the emitter terminal 50, the support system 8 matches the sell request and the buy request, thereby enabling movement of a token(s) from the user wallet 40 to the emitter wallet 60 while eliminating temporary storage of the token(s) in the manager wallet 20. Therefore, the support system 8 can further reduce the burden of the manager wallet 20.

### 5. Examples of Effects

In the consumption amount calculation system 70, the first parameter related to carbon dioxide supplied via the first supply passage 91 can be detected by the first detection section 71, and the second parameter related to carbon dioxide contained in the product produced by the carbon dioxide utilization device 80 can be detected by the second detection section 72. The consumption amount calculation method realized by the consumption amount calculation system 70 can more accurately calculate "the amount of carbon dioxide consumed in the carbon dioxide utilization device 80" by reflecting the first parameter obtained at the passage through which carbon dioxide flows before being supplied to the carbon dioxide utilization device 80 and the second parameter based on the product obtained as a result of supply of carbon dioxide to the carbon dioxide utilization device 80.

Specifically, the consumption amount calculation method realized by the consumption amount calculation system 70 can further accurately calculate "the amount of carbon dioxide consumed in the carbon dioxide utilization device 80" on the basis of the difference between "the carbon dioxide supply amount determined on the basis of the first parameter" and "the amount of carbon dioxide remaining in the product determined on the basis of the second parameter."

Since the consumption amount calculation system 70 includes the record section (the data processing unit 75) which hashes at least one of the first parameter and the second parameter and records the hashed parameter(s) in the blockchain, at least one of the first parameter and the second parameter can be managed by using the blockchain. Therefore, the consumption amount calculation system 70 can enhance the function of preventing unauthorized manipulation of the parameter(s), and can operate the system while ensuring the reliability of the parameter(s).

Since the consumption amount calculation system 70 has the record section (the data processing unit 75) which hashes the program changing history and records the hashed program changing history in the blockchain, the program changing history can be managed by using the blockchain. Therefore, the consumption amount calculation system 70 can enhance the function of preventing unauthorized manipulation of the program. Thus, this consumption amount calculation system 70 can operate the system while ensuring the reliability of the program.

When the consumption amount calculation system 70 generates current data containing at least one of the first parameter and the second parameter, the consumption amount calculation system 70 can compare the current data with the data recorded in the cloud before generation of the current data and verify the current data.

The emission trading support system 8 can provide an incentive to a user contributing to consumption (reduction) of carbon dioxide by sending tokens to the user wallet 40. Furthermore, in the case where carbon dioxide has been consumed in the utilization device 80, the emission trading support system 8 can more accurately calculate the amount of consumed carbon dioxide and more properly issue a token(s) on the basis of the calculated consumption amount.

### <Second embodiment>

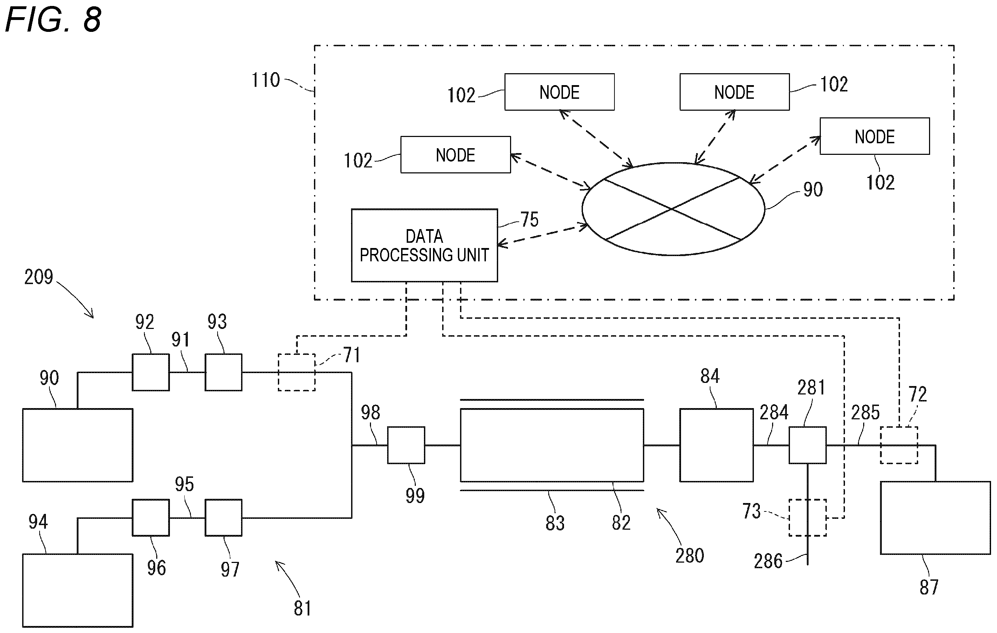

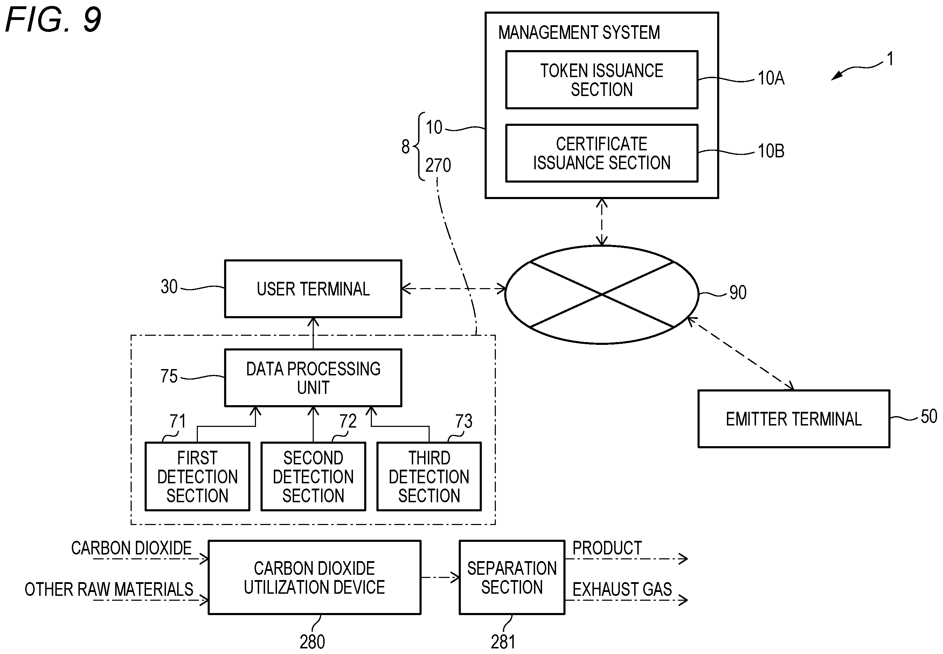

The following description relates to a carbon dioxide consumption amount calculation system 270 according to a second embodiment shown in FIGS. 8 and 9 and a carbon dioxide utilization system 209 which uses this. The carbon dioxide consumption amount calculation system 270 will be also referred to simply as the consumption amount calculation system 270. The carbon dioxide utilization system 209 will be also referred to simply as the utilization system 209.

The utilization system 209 to which the consumption amount calculation system 270 of the second embodiment is applied differs from the above-described utilization system 9 (FIG. 1) in that a carbon dioxide utilization device 280 is used instead of the carbon dioxide utilization device 80 and a third detection section 73 is used. Since the utilization system 209 is the same as the above-described utilization system 9 (FIG. 1) in other points, configurational elements of the utilization system 209 identical with those of the utilization system 9 (FIG. 1) are denoted by the same reference signs as the utilization system 9 and their detailed descriptions are omitted. The carbon dioxide utilization device 280 will be also referred to simply as the utilization device 280.

Although the above-described emission trading support system 8 (FIG. 4, etc.) is realized by providing the consumption amount calculation system 70 according to the first embodiment, the emission trading support system 8 may be realized by providing the consumption amount calculation system 270 according to the second embodiment instead of the consumption amount calculation system 70. In this case, the emission trading support system 8 differs from the above-described emission trading support system 8 (FIG. 4, etc.) only in that the consumption amount is calculated by the consumption amount calculation system 270 instead of the consumption amount calculation system 70 and is the same as the above-described emission trading support system 8 (FIG. 4, etc.) in other points.

In the utilization system 209 shown in FIG. 8, the configuration and function of the raw material feed section 81 are identical with those of the raw material feed section 81 of the above-described utilization system 9 (FIG. 1).

The utilization device 280 is a device which produces a carbon dioxide-originating product by using, as a raw material, carbon dioxide supplied from the carbon dioxide supply section 90. The utilization device 280 includes a reaction vessel 82, a heater 83, a condenser 84, a downstream flow passage 284, a separation section 281, a product passage 285, an exhaust gas passage 286, a tank 87, etc. In the example described below, the utilization device 280 is configured as a methanation device which causes the plurality of types of raw materials (raw materials containing carbon dioxide) fed by the raw material feed section 81 to react with each other, thereby generating methane as a carbon dioxide-originating product.

The reaction vessel 82 is a vessel through which the gas mixture flowing thereinto through the merging passage 98 flows and in which a reaction occurs. The reaction vessel 82 has an appropriately set pressure and is heated by the heater 83, whereby, for example, the Sabatier reaction and the water gas shift reaction represented by the following chemical reaction formula are conducted, and thus, methane is produced.

3CO₂ + 6H₂ → CH₄ + 2CO + 4H₂O

The product produced in the reaction vessel 82 is cooled by the condenser 84, whereby the product is separated to water and a gas containing methane. The gas containing methane is fed to the downstream flow passage 284. "The gas containing methane" separated by the condenser 84 may contain hydrogen and carbon dioxide of the raw material gas.

The separation section 281 is provided in the downstream flow passage 284 on the downstream side of the condenser 84. In the representative example, the separation section 281 is a separation membrane for selectively separating CH₄ from the gas (the gas containing methane) passing through the downstream flow passage 284. The separation section 281 separates the gas passing through the downstream flow passage 284 to a fraction thereof mainly composed of CH₄ and a remaining fraction thereof and causes CH₄ to flow into the product passage 285 and the remaining fraction (a fraction of the gas other than the fraction flowing into the product passage 285) to flow into the exhaust gas passage 286. The gas fed into the product passage 285 may be composed of only CH₄ or may contain gases other than CH₄ in small amounts. The methane having flowed through the product passage 285 is compressed by, for example, an unillustrated compressor and is stored in the tank 87 similar to that shown in FIG. 1.

In this example as well, the first detection section 71 measures the concentrations (volume ratios) of components (CO₂, H₂O, etc.) contained in the gas flowing through the first supply passage 91 and determines the flow rate of the gas flowing through the first supply passage 91 by the same method as in the first embodiment. For example, in the case where the flow rate of the gas flowing through the first supply passage 91, which is measured by the flowmeter contained in the first detection section 71, is 90 L/min and the result of measurement by the gas chromatography contained in the first detection section 71 shows that, in the gas flowing through the first supply passage 91, the volume ratio of CO₂ is 33 vol% and the volume ratio of H₂O is 0 vol%, it is determined that the volume ratio of H₂ is 67 vol%. Therefore, it is possible to determine, on the basis of the above-mentioned flow rate and the volumes ratios of the respective gases, that the flow rate of CO₂ flowing through the first supply passage 91 is 30 L/min, the flow rate of H₂ flowing through the first supply passage 91 is 60 L/min, and the flow rate of H₂O flowing through the first supply passage 91 is 0 L/min. In this example, the flow rate X1 of CO₂ flowing through the first supply passage 91 corresponds to one example of the first parameter.

The second detection section 72 is a device which detects a second parameter related to carbon dioxide contained in the product in the product passage 285 provided on the downstream side of the utilization device 280 (the gas passing through the product passage 285). The second detection section 72 includes a flowmeter and a gas chromatography and measures the volume ratios of CH₄, CO₂, CO, and H₂O in the gas flowing through the product passage 285 and the gas flow rate of the gas flowing through the product passage 285. For example, in the case where the flow rate of the gas flowing through the product passage 285, which is measured by the flowmeter contained in the second detection section 72, is 5 L/min and the result of measurement by the gas chromatography contained in the second detection section 72 shows that, in the gas flowing through the product passage 285, the volume ratio of CO₂ is 0 vol%, the volume ratio of H₂O is 0 vol%, the volume ratio of CH₄ is 100 vol%, and the volume ratio of CO is 0 vol%, it is possible to determine that the flow rate of CO₂ flowing through the product passage 285 is 0 L/min, the flow rate of H₂O flowing through the product passage 285 is 0 L/min, the flow rate of CH₄ flowing through the product passage 285 is 5 L/min, and the flow rate of CO flowing through the product passage 285 is 0 L/min. In this example, the flow rate X2 of CO₂ flowing though the product passage 285 corresponds to one example of the second parameter.

The third detection section 73 is a device which detects a third parameter related to "carbon dioxide contained in exhaust gas" in the exhaust gas passage 286 provided on the downstream side of the utilization device 280 such that exhaust gas (i.e., gas components other than the product) flows through the exhaust gas passage 286. Specifically, the third detection section 73 measures the volume ratios of CO₂, CO, and H₂O in the gas flowing through the exhaust gas passage 286 and the gas flow rates of the gas flowing through the exhaust gas passage 286. For example, in the case where the flow rate of the gas flowing through the exhaust gas passage 286, which is measured by the flowmeter contained in the third detection section 73, is 75 L/min and the result of measurement by the gas chromatography contained in the third detection section 73 shows that, in the gas flowing through the exhaust gas passage 286, the volume ratio of CO₂ is 20 vol%, the volume ratio of H₂O is 27 vol%, the volume ratio of CH₄ is 0 vol%, and the volume ratio of CO is 13 vol%, it is possible to determine that the flow rate of CO₂ flowing through the exhaust gas passage 286 is 15 L/min, the flow rate of H₂O flowing through the exhaust gas passage 286 is 20 L/min, the flow rate of CH₄ flowing through the exhaust gas passage 286 is 0 L/min, the flow rate of CO flowing through the exhaust gas passage 286 is 10 L/min, and the flow rate of H₂ flowing through the exhaust gas passage 286 is 30 L/min. In this example, the flow rate X3 of CO₂ flowing though the exhaust gas passage 286 corresponds to one example of the third parameter. Notably, CO flowing through the exhaust gas passage 286 may be used as a fuel or the like.

The data processing unit 75 (the calculation section) calculates the consumption amount on the basis of the first parameter, the second parameter, and the third parameter. For example, the data processing unit 75 may calculate, as the consumption amount of carbon dioxide, a value (X1 - X2 - X3) by subtracting the flow rate X3 of CO₂ flowing through the exhaust gas passage 286 from the difference (X1 - X2) between the flow rate X1 of CO₂ flowing through the first supply passage 91 and the flow rate X2 of CO₂ flowing through the product passage 285. In the above-described specific example, the flow rate X1 of CO₂ flowing through the first supply passage 91 is 30 L/min, the flow rate X2 of CO₂ flowing through the product passage 285 is 0 L/min, and the flow rate X3 of CO₂ flowing through the exhaust gas passage 286 is 15 L/min. Therefore, in this case, 15 L/min, which corresponds to the value of X1 - X2 - X3, can be calculated as the consumption amount (specifically, consumption amount per unit time) of carbon dioxide consumed in the utilization device 80.

As described above, in the case where the above-described product (methane) and exhaust gas (gas components other than the above-described product) are produced in the utilization device 280, the consumption amount calculation system 270 according to the second embodiment can detect the third parameter related to carbon dioxide contained in the exhaust gas by the third detection section 73. Thus, the consumption amount calculation system 270 can more accurately calculate "the amount of carbon dioxide consumed in the carbon dioxide utilization device 280" by reflecting the first parameter obtained at the passage through which carbon dioxide flows before being supplied to the utilization device 280, the second parameter based on the above-described product (methane) obtained as a result of supply of carbon dioxide to the utilization device 280, and the third parameter based on carbon dioxide contained in the remaining exhaust gas.

### <Other embodiments>

The present disclosure is not limited to the embodiments described by the above description with reference to the drawings. The features of the above-described embodiments and embodiments which will be described below can be combined in any manner so long as no contradiction occurs. Also, any of the features of the above-described embodiments and the embodiments which will be described below can be omitted so long as it is not clearly described as essential. Furthermore, the features of the above-described embodiments may be changed as follows.

In the above-described embodiments, the carbon dioxide utilization devices 80 and 280 are methanation devices. However, the carbon dioxide utilization devices 80 and 280 are not limited thereto and may be any device so long as the device can produce a carbon dioxide-originating product, which is different from carbon dioxide, by using carbon dioxide as a raw material. For example, the carbon dioxide utilization devices 80 and 280 may be devices which produce ethanol. In this case, the raw material feed section 81 may be configured in the same manner as in FIGS. 1 and 8 so as to feed hydrogen and carbon dioxide as raw materials, and ethanol is produced in the reaction vessel 82 by the chemical reaction represented by the following chemical reaction formula.

6H₂ + 2CO₂ → CH₃CH₂OH + 3H₂O

For example, in the example in which the configuration of FIG. 1 is applied to ethanol production, as in the first embodiment, the first detection section 71 measures the flow rate of the gas flowing through the first supply passage 91 (gas flow rate) and the volume ratios of CO₂ and H₂O in the gas flowing through the first supply passage 91. For example, in the case where the flow rate of the gas flowing through the first supply passage 91 is 80 L/min and the result of measurement by the gas chromatography contained in the first detection section 71 shows that, in the gas flowing through the first supply passage 91, the volume ratio of CO₂ is 25 vol% and the volume ratio of H₂O is 0 vol%, it is determined that the volume ratio of H₂ is 75 vol%. Therefore, it is possible to determine, on the basis of the above-mentioned flow rate and the volumes ratios of the respective gases, that the flow rate of CO₂ flowing through the first supply passage 91 is 20 L/min, the flow rate of H₂ flowing through the first supply passage 91 is 60 L/min, and the flow rate of H₂O flowing through the first supply passage 91 is 0 L/min. In this example, the flow rate X1 of CO₂ flowing through the first supply passage 91 corresponds to one example of the first parameter.

The second detection section 72 detects the flow rate of the gas flowing through the downstream flow passage 85. For example, in the case where the flow rate of the gas flowing through the downstream flow passage 85, which is measured by the flowmeter contained in the second detection section 72, is 40 L/min and the result of measurement by the gas chromatography contained in the second detection section 72 shows that, in the gas flowing through the downstream flow passage 85, the volume ratio of CO₂ is 0 vol%, the volume ratio of H₂O is 75 vol%, the volume ratio of ethanol is 25 vol%, and the volume ratio of CO is 0 vol%, it is possible to determine that the flow rate of CO₂ flowing through the downstream flow passage 85 is 0 L/min, the flow rate of H₂O flowing through the downstream flow passage 85 is 30 L/min, the flow rate of ethanol flowing through the downstream flow passage 85 is 10 L/min, and the flow rate of CO flowing through the downstream flow passage 85 is 0 L/min. In this example, the flow rate X2 of CO₂ flowing though the downstream flow passage 85 corresponds to one example of the second parameter.

In this example as well, the data processing unit 75 can calculate the amount of carbon dioxide consumed in the utilization device 80 on the basis of the first parameter and the second parameter described above. The data processing unit 75 can determine the amount of carbon dioxide consumed in the utilization device 80 by calculating, for example, the difference (X1 - X2) between the first parameter and the second parameter.

In the first embodiment and other embodiments obtained by modifying the first embodiment, the difference (X1 - X2) between the first parameter and the second parameter itself can be used as the amount of carbon dioxide consumed in the utilization device 80. However, a value obtained by performing a certain correction calculation on the difference (X1 - X2) between the first parameter and the second parameter may be used as the amount of carbon dioxide consumed in the utilization device 80. Similarly, in the second embodiment as well, a value obtained by performing a certain correction calculation on the above-described value (X1 - X2 - X3) may be used as the amount of carbon dioxide consumed in the utilization device 280.

In the above-described embodiments, the consumption amount calculation system has a configuration which does not include "the supply section" and "the carbon dioxide utilization device." However, the consumption amount calculation system is not limited to this configuration, and one or both of "the supply section" and "the carbon dioxide utilization device" may be included in the consumption amount calculation system. For example, in the first embodiment, one or both of the carbon dioxide supply section 90 and the carbon dioxide utilization device 80 may be included in the consumption amount calculation system 70 as its configurational elements.

In the above-described embodiments, the first parameter is the above-mentioned flow rate X1. However, the first parameter may be any parameter that enables determination of the amount of CO₂ flowing through the first supply passage 91. For example, the first parameter may be the flow rate of the gas flowing through the first supply passage 91 and the volume ratios of CO₂, H₂O, H₂ in the gas flowing through the first supply passage 91. The first parameter is not required to be the flow amount per unit time and may be the volume of carbon dioxide fed into the reaction vessel 82 within a predetermined period of time or any of other indexes (pressure, temperature, etc.) which can determine the volume.

In the first embodiment, the second parameter is the above-mentioned flow rate X2. However, the second parameter may be any parameter that enables determination of the amount of CO₂ flowing through the downstream flow passage 85. For example, the second parameter may be the flow rate of the gas flowing through the downstream flow passage 85 and the volume ratios of CO₂, H₂O, H₂ in the gas flowing through the downstream flow passage 85. The second parameter is not required to be the flow amount per unit time and may be the volume of carbon dioxide fed into the tank 87 within a predetermined period of time or any of other indexes (pressure, temperature, etc.) which can determine the volume.

In the second embodiment, the second parameter is the above-mentioned flow rate X2. However, the second parameter may be any parameter that enables determination of the amount of CO₂ flowing through the product passage 285. For example, the second parameter may be the flow rate of the gas flowing through the product passage 285 and the volume ratios of CO₂, H₂O, H₂ in the gas flowing through the product passage 285. Alternatively, the second parameter may be the volume of carbon dioxide fed into the tank 87 within a predetermined period of time or any of other indexes (pressure, temperature, etc.) which can determine the volume.

In the second embodiment, the third parameter may be any parameter that enables determination of the amount of CO₂ flowing through the exhaust gas passage 286. For example, the second parameter may be the flow rate of the gas flowing through the exhaust gas passage 286 and the volume ratios of CO₂, H₂O, H₂ in the gas flowing through the exhaust gas passage 286. Alternatively, the second parameter may be the volume of carbon dioxide having passed through the exhaust gas passage 286 within a predetermined period of time or any of other indexes (pressure, temperature, etc.) which can determine the volume.

In the above-described embodiments, the support system 8 is configured by the management system 10 and the manager wallet 20. However, in the support system 8 in ether of the embodiments, one or both of the user terminal 30 and the emitter terminal 50 may be contained in the support system 8. Alternatively, one or both of the user wallet 40 and the emitter wallet 60 may be contained in the support system 8.

Notably, the embodiments disclosed this time should be considered to be illustrative and not to be restrictive in all aspects. The scope of the present invention is not limited to the embodiments disclosed this time, and it is intended that the present invention encompasses all modifications within the range shown by the claims and the range of equivalents of the claims.

### REFERENCE SIGNS LIST

8: emission trading support system
9: carbon dioxide utilization system
10: management system
10A: token issuance section
20: manager wallet
30: user terminal
40: user wallet
70, 270: consumption amount calculation system
71: first detection section
72: second detection section
73: third detection section
75: data processing unit (calculation section, record section, changing history record section, data record section, comparison section)
80, 280: carbon dioxide utilization device
90: carbon dioxide supply section (supply section)
91: first supply passage (raw material supply passage)
100: blockchain
110: blockchain
270: consumption amount calculation system
281: separation section
285: product passage
286: exhaust gas passage

## Claims

1. A carbon dioxide consumption amount calculation system comprising:
a first detection section disposed in a raw material supply passage between a supply section serving as a carbon dioxide supply source and a carbon dioxide utilization device which produces a carbon dioxide-originating product by using, as a raw material, carbon dioxide supplied from the supply section, the first detection section detecting a first parameter related to carbon dioxide supplied via the raw material supply passage;
a second detection section which detects a second parameter related to carbon dioxide contained in the product produced by the carbon dioxide utilization device; and
a calculation section which calculates the consumption amount of carbon dioxide consumed in the carbon dioxide utilization device on the basis of the first parameter and the second parameter.

2. A carbon dioxide consumption amount calculation system according to claim 1,
wherein the calculation section calculates the consumption amount on the basis of the difference between the amount of supplied carbon dioxide determined on the basis of the first parameter and the amount of carbon dioxide remaining in the product determined on the basis of the second parameter.

3. A carbon dioxide consumption amount calculation system according to claim 1 or 2,
wherein the second detection section detects the second parameter related to carbon dioxide contained in the product in a product passage provided on the downstream side of the carbon dioxide utilization device,
wherein the carbon dioxide consumption amount calculation system further comprises a third detection section which detects a third parameter related to carbon dioxide contained in exhaust gas in an exhaust gas passage provided on the downstream side of the carbon dioxide utilization device such that the exhaust gas other than the product flows through the exhaust gas passage, and
wherein the calculation section calculates the consumption amount on the basis of the first parameter, the second parameter, and the third parameter.

4. A carbon dioxide consumption amount calculation system according to any one of claims 1 to 3, comprising a record section which hashes at least one of the first parameter and the second parameter and records the hashed parameter(s) in a blockchain.

5. A carbon dioxide consumption amount calculation system according to any one of claims 1 to 4,
wherein the calculation section includes a program for calculating the consumption amount on the basis of the first parameter and the second parameter, and
wherein the carbon dioxide consumption amount calculation system further comprises a changing history record section which hashes a changing history of the program and records the hashed changing history in a blockchain.

6. A carbon dioxide consumption amount calculation system according to any one of claims 1 to 5, comprising:
a data record section which records data in a cloud, the data containing at least one of the first parameter and the second parameter; and
a comparison section which operates, when at least one of the first detection section and the second detection section generates current data containing at least one of the first parameter and the second parameter, so as to compare the current data with data recorded in the cloud before generation of the current data.

7. A carbon dioxide consumption amount calculation method comprising:
calculating the consumption amount of carbon dioxide consumed in a carbon dioxide utilization device on the basis of a first parameter and a second parameter related to carbon dioxide,
the first parameter being related to carbon dioxide supplied via a raw material supply passage between a supply section serving as a carbon dioxide supply source and the carbon dioxide utilization device which produces a carbon dioxide-originating product by using, as a raw material, carbon dioxide supplied from the supply section, and
the second parameter being related to carbon dioxide contained in the product produced by the carbon dioxide utilization device.

8. A carbon dioxide consumption amount calculation method according to claim 7,
wherein the consumption amount is calculated on the basis of the difference between the amount of supplied carbon dioxide determined on the basis of the first parameter and the amount of carbon dioxide remaining in the product determined on the basis of the second parameter.

9. A carbon dioxide consumption amount calculation method according to claim 7,
wherein the consumption amount is calculated on the basis of the first parameter, the second parameter, and a third parameter related to carbon dioxide contained in exhaust gas in an exhaust gas passage provided on the downstream side of the carbon dioxide utilization device such that the exhaust gas other than the product flows through the exhaust gas passage.

10. A carbon dioxide emission trading support system comprising:
a management system having a token issuance section for issuing a token; and
a manager wallet associated with the management system,
wherein, in the case where a user account and a user wallet are associated with each other, and a consumption signal for notifying an amount of consumption of carbon dioxide is sent from a user terminal associated with the user account to the management system, the token issuance section issues the token on the basis of the consumption signal, and the management system instructs storage of the issued token in the user wallet, and
wherein the consumption signal is generated and sent on the basis of the consumption amount calculated by the carbon dioxide consumption amount calculation method according to any one of claims 7 to 9.
